# EUROPEAN PATENT APPLICATION

(11) **EP 4 258 282 A1**
(43) Date of publication of application: **11.10.2023**
(21) Application number: 21917820.9
(22) Date of filing: 29.03.2021
(51) Int. Cl.: G16H 50/70, G16H 30/40, G16H 30/20, G16H 50/20, G06N 3/08, G06N 3/04

(54) **METHOD FOR ANALYZING OUTPUT OF NEURAL NETWORK, AND SYSTEM THEREFOR**

(30) Priority: 07.01.2021 KR 20210001913
(71) Applicant: Deep Bio Inc., Seoul 08380 (KR)
(72) Inventor: CHO, Min Ah, Seoul 04776 (KR); CHO, Joon Young, Seoul 08394 (KR); KWAK, Tae Yeong, Seoul 05119 (KR); KIM, Sun Woo, Seongnam-si, Gyeonggi-do 13599 (KR)
(74) Representative: Flügel Preissner Schober Seidel
(86) International application number: PCT/KR2021/003823
(87) International publication number: WO 2022/149658

(57) **Abstract**

Disclosed are a method for analyzing an output of a neural network, and a system therefor. According to one aspect of the present invention, a method for analyzing an output of a neural network that analyzes an output result of a neural network trained so as to output a disease expression probability for each pixel of a biological image, comprises: a step in which, depending on whether an output result value of the neural network for each pixel is equal to or greater than an optimal reference value, an output analysis system determines the optimal output result value which is a reference value for detecting whether a disease is expressed in the corresponding pixel; a step of determining an optimal cut-off value for determining whether a detected lesion site is effective with respect to a detected lesion in a biological image which is specified when the expression of a disease for each pixel is detected in a biological image by using the optimal reference value determined by the output analysis system; and a step of, when the output analysis system receives, from the neural network, an output result corresponding to a diagnostic biometric image to be diagnosed, performing an output analysis on the output result by using the determined optimal reference value and the optimal cut-off value.

## Description

### Technical Field

The present disclosure relates to a method of analyzing an output of a neural network and a system therefor. More specifically, the present disclosure relates to a method that enables more accurate diagnosis of a disease by effectively analyzing an output result output by a neural network that is trained for diagnosis of the disease, and a system therefor.

### Background Art

One of the main tasks performed by the pathology or pathology department is to perform diagnosis to determine the condition or symptom of a specific disease by reading a patient's biometric image. This diagnosis is a method that relies on the experience and knowledge of skilled medical personnel for a long time.

Recently, thanks to the development of machine learning, attempts to automate tasks such as recognizing or classifying images by computer systems have been actively made. In particular, attempts have been made to automate the diagnosis that has been performed by skilled medical personnel using a neural network (e.g., a deep learning method using a convolutional neural network (CNN)), which is a type of machine learning.

In particular, diagnosis through deep learning using the neural network (e.g., CNN) does not simply automate the experience and knowledge of conventionally skilled medical personnel, but in that it finds the characteristic elements through self-learning and derives the desired answer, in some cases, the characteristics of disease factors that the skilled medical personnel were not aware of may be found in images.

In general, the diagnosis of diseases through the neural network using biometric images involves the use of fragments of biometric images, that are, patches (also referred to as tiles). In other words, for the tile, the skilled medical professional annotates a state of a specific disease (e.g., whether cancer is developed) and trains the neural network using a plurality of these annotated tiles as training data.

The neural network trained thereby may be implemented to output the probability that the pixel is included in an area where the disease is developed, in other words, a lesion area for each pixel.

This has significance as information to estimate the likelihood of inclusion in the lesion area for each pixel, but since it does not conclusively determine whether it is included in the lesion area by itself, an additional output analysis step may be required to decide whether the information on the likelihood of pixel units output by the neural network is included in the actual lesion area.

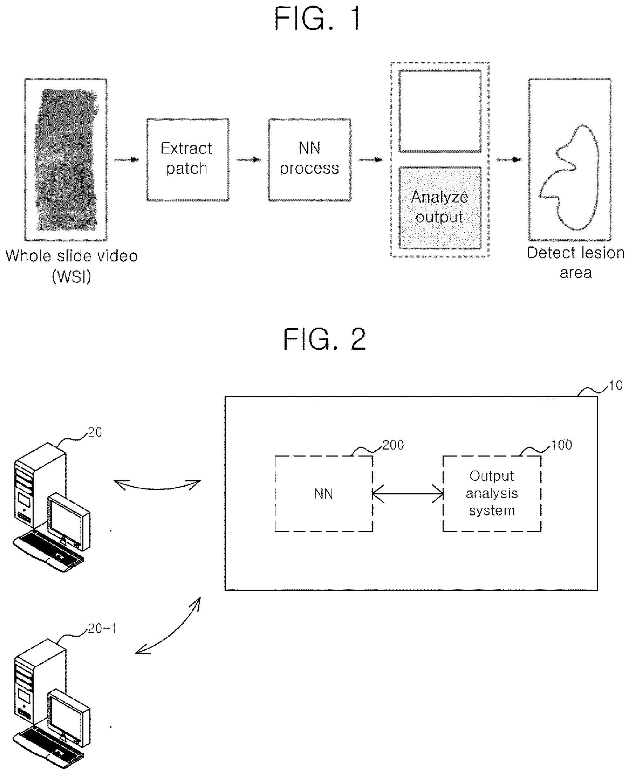

FIG. 1 is a diagram that outlines a process of diagnosing a disease using a conventional neural network.

Referring to FIG. 1, a neural network processing process may be performed, in that a biometric image (also referred to as a whole slide image) is divided into partial images (also referred to as patch images) in a certain size, and the neural network trained for each divided partial image outputs the probability of being included in the lesion area for each pixel in the patch.

Then, by undergoing a process of analyzing the output result output by the neural network for each patch, the lesion area in the biometric image is finally detected.

In the process of analyzing the output results, it is apparent that a process of combining the output results for each patch back into a range of slides.

In general, the process of analyzing the output result may be analyzed to be included in the lesion area if the degree of probability for each pixel is greater than or equal to 0.5, and not to be included therein if it is less than 0.5, and the training may be performed in consideration of the analysis on the basis of these assumptions in the training of the neural network.

This method may be optimal when there is no error at all in the annotation information used for training of the neural network, and the total size of the lesion area in the biometric image used for training and the total size of the tissue area not included in the lesion are similar. However, since the construction of the annotation information is made according to the subjective determination of the pathologist, there may be some inconsistencies and errors in the constructed annotation information, and the neural network trained to learn this may therefore determine that some lesion areas are not lesion areas or misjudge some non-lesion areas as lesion areas, such that the conventional method alone has a problem in that the accuracy for the lesion area detected through the output analysis process is reduced inevitably.

Therefore, a technical idea is required to make the output analysis process to determine whether to be included in the lesion area for the output result of the neural network for each pixel more effective with increased accuracy.

### [Prior Art Document]

### [Patent Document]

(Patent Document 1) Korean Patent Application Publication No. 10-2019-0143510 "Two-face disease diagnosis system and method therefor"

### Disclosure of the Invention

### Technical Goals

A technical object to be achieved by the present disclosure is to provide a method of analyzing an output result effectively with high accuracy based on an output result of a neural network trained to output the probability that a disease is developed for each pixel, in other words, the probability of being included in a lesion area, and a system therefor.

### Technical Solutions

According to one aspect of the present disclosure, a method of analyzing an output of a neural network that analyzes an output result of the neural network trained to output a probability of disease development by each pixel of a biometric image includes determining, by an output analysis system, an optimal reference value, which is a criterion to detect whether a disease is developed in each pixel depending on whether an output result value of the neural network for the pixel is greater than or equal to the optimal reference value, determining, by the output analysis system, an optimal cut-off value to determine, for a detected lesion area on the biometric image that is specified when whether the diseases is developed for each pixel is detected for the biometric image using the determined optimal reference value, whether the detected lesion area is valid, and, when receiving an output result corresponding to a diagnostic biometric image to be diagnosed from the neural network, performing, by the output analysis system, output analysis on the output result using the determined optimal reference value and the determined optimal cut-off value.

The determining of the optimal reference value may include obtaining, by the output analysis system, an output value for each pixel of each of a plurality of biometric images using the neural network, setting, by the output analysis system, a plurality of candidate reference values and calculating, for each of the set candidate reference values, a degree of concordance when the disease development is detected by applying each of the candidate reference values, wherein the degree of concordance is determined by a degree of which a detection result detected by applying the candidate reference value for each pixel and an annotated result are concordant, and determining, by the output analysis system, the optimal reference value based on the calculated degree of concordance of each of the candidate reference values.

The calculating of the degree of concordance when whether the disease is developed is determined by applying each of the candidate reference values may include calculating the degree of concordance using at least one of accuracy, Intersection over Union (IoU), or Dice Similarity Coefficient (DSC) for all of the plurality of biometric images; or an average of accuracy, Intersection over Union (IoU), or Dice Similarity Coefficient (DSC) of each of the plurality of biometric images, wherein the accuracy may be defined as (TP + TN)/(TP + TN + FP + FN), the IoU as TP/(TP + FP + FN), and the DSC as 2×TP/(2 × TP + FP + FN), and the TP may be the number of pixels included in both a first lesion area detected through the output analysis system and an annotated second lesion area, the FN may be the number of pixels included only in the second lesion area, the FP may be the number of pixels included only in the first lesion area, and the TN may be the number of pixels not included in both the first lesion area and the second lesion area.

The determining of the optimal cut-off value to determine whether the detected lesion area is valid may include obtaining, by the output analysis system, an output value for each pixel of each of a plurality of biometric images using the neural network, detecting, by the output analysis system, at least one first lesion area included in each of the plurality of biometric images using the obtained output value for each pixel of each of the plurality of biometric images and the optimal reference value to obtain the number of pixels included in the first lesion area, and determining, based on the number of pixels for each of the detected at least one first lesion area and whether the disease is developed actually, the optimal cut-off value which is a criterion to determine the validity of the detected first lesion area.

The determining of the optimal cut-off value which is the criterion to determine the validity of the detected first lesion area based on the number of pixels included in a second lesion area which is an annotated lesion area for each of the plurality of biometric images and the first lesion area may include determining, by the output analysis system, the optimal cut-off value using any one of linear regression, logistic regression, or support vector machine.

The determining of the optimal cut-off value which is the criterion to determine the validity of the detected first lesion area based on the number of pixels included in a second lesion area which is an annotated lesion area for each of the plurality of biometric images and the first lesion area may include determining, by the output analysis system, an initial optimal cut-off value, determining, by the output analysis system, whether the initial optimal cut-off value predetermined satisfies a predetermined minimum sensitivity or a predetermined minimum specificity, and, if the determination result is satisfied, determining the initial optimal cut-off value as the optimal cut-off value and, if unsatisfied, searching for the optimal cut-off value while sequentially changing the initial optimal cut-off value by a predetermined unit until the minimum sensitivity or the minimum specificity is satisfied.

The method may further include determining, by the output analysis system, a reference value for each pixel by each pixel using the determined optimal reference value, wherein the output analysis system may detect whether the disease is developed for each pixel for the diagnostic biometric image to be diagnosed using the determined reference value for each pixel, and the reference value for each pixel may be determined by correcting the optimal reference value using an output value of the neural network for at least one predetermined peripheral pixel based on a target pixel.

A method of analyzing an output of a neural network according to another embodiment includes obtaining, by the output analysis system, an output result value of the neural network by each pixel for a diagnostic biometric image to be diagnosed, and detecting, by the output analysis system, whether a disease is developed for each pixel by comparing the obtained output result value with a predetermined reference value, wherein the reference value is determined differently for each pixel and determined by correcting entire reference values determined in a predetermined manner for the entire diagnostic biometric image using the output result value of the neural network for at least one peripheral pixel predetermined based on a target pixel.

The above method may be implemented by a computer program.

An output analysis system of a neural network 200 according to one aspect includes a processor and a memory in which the program is stored, wherein the processor is configured to run the program to determine an optimal reference value, which is a criterion to detect whether a disease is developed in each pixel depending on whether an output result value of the neural network for the pixel is greater than or equal to the optimal reference value, determine an optimal cut-off value to determine, for a detected lesion area on the biometric image that is specified when whether the diseases is developed for each pixel is detected for the biometric image using the determined optimal reference value, whether the detected lesion area is valid, and, when receiving an output result corresponding to a diagnostic biometric image to be diagnosed from the neural network, perform output analysis on the output result using the determined optimal reference value and the determined optimal cut-off value.

The processor may be configured to run the program to obtain an output value for each pixel of each of a plurality of biometric images using the neural network, set a plurality of candidate reference values and calculate, for each of the set candidate reference values, a degree of concordance when the disease development is detected by applying each of the candidate reference values, wherein the degree of concordance is determined by a degree of which a detection result detected by applying the candidate reference value for each pixel and an annotated result are concordant, and determine the optimal reference value based on the calculated degree of concordance of each of the candidate reference values.

The processor may be configured to run the program to obtain an output value for each pixel of each of a plurality of biometric images using the neural network, detect at least one first lesion area included in each of the plurality of biometric images using the obtained output value for each pixel of each of the plurality of biometric images and the optimal reference value to obtain the number of pixels included in the first lesion area, and, based on the number of pixels for each of the detected at least one first lesion area and whether the disease is developed actually, determine the optimal cut-off value which is a criterion to determine the validity of the detected first lesion area.

The processor may be configured to run the program to determine an initial optimal cut-off value, determine whether the initial optimal cut-off value predetermined satisfies a predetermined minimum sensitivity or a predetermined minimum specificity, and if the determination result is satisfied, determine the initial optimal cut-off value as the optimal cut-off value and, if unsatisfied, search for the optimal cut-off value while sequentially changing the initial optimal cut-off value by a predetermined unit until the minimum sensitivity or the minimum specificity is satisfied.

The processor may be configured to run the program to determine a reference value for each pixel by each pixel using the determined optimal reference value and detect whether the disease is developed for each pixel for the diagnostic biometric image to be diagnosed using the determined reference value for each pixel, wherein the reference value for each pixel may be determined by correcting the optimal reference value using an output value of the neural network for at least one predetermined peripheral pixel based on a target pixel.

According to another aspect, an output analysis system of a neural network according to the technical idea of the present disclosure includes a processor and a memory in which a program is stored, wherein the processor is configured to run the program to obtain an output result value of the neural network by each pixel for a diagnostic biometric image to be diagnosed, and detect whether a disease is developed for each pixel by comparing the output result value with a predetermined reference value while the reference value may be determined differently for each pixel and determined by correcting entire reference values determined in a predetermined manner for the entire diagnostic biometric image using the output result value of the neural network for at least one peripheral pixel predetermined based on a target pixel.

According to another aspect of the present disclosure, there is provided a computer program which is installed in a data processing device and recorded on a non-transitory medium for implementing the above-described method.

### Advantageous Effects

According to the technical idea of the present disclosure, by effectively determining a reference value for determining whether a pixel is included in a lesion area for each pixel such that the lesion area detected according to the analysis result of an output of a neural network can be consistent with an actual lesion area (annotated lesion area) as much as possible, there is an effect of increasing the accuracy in the diagnosis of a disease according to an output analysis result compared to a case in which a pre-arbitrarily determined reference value is used.

In addition, by defining and utilizing a cut-off value for an analyzed lesion area using the reference value determined thereby, there is an effect that the output of the neural network may be analyzed, in that whether the lesion area is detected can be consistent with the presence of the disease in an actual sample as much as possible only with a sample unit (biometric imaging unit).

### Brief Description of Drawings

In order to more fully understand the drawings cited in the detailed description of the present disclosure, a brief description of each drawing is provided.
FIG. 1 is a diagram for outlining a process of detecting a lesion area using a conventional neural network.
FIG. 2 is a diagram for describing a schematic system configuration for implementing a method of analyzing an output of a neural network in accordance with an embodiment of the present disclosure.
FIG. 3 is a diagram for describing a schematic configuration of an output analysis system of a neural network in accordance with an embodiment of the present disclosure.
FIG. 4 is a flowchart for outlining a concept of analyzing an output result of a neural network in accordance with an embodiment of the present disclosure.
FIG. 5 is a flowchart for describing a way of determining an optimal reference value in accordance with an embodiment of the present disclosure.
FIG. 6 is a flowchart for describing a way of determining an optimal cut-off value in accordance with an embodiment of the present disclosure.
FIG. 7 is a flowchart for describing a way of determining a reference value for each pixel in accordance with an embodiment of the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present disclosure, if it is determined that a detailed description of related known technologies may obscure the gist of the present disclosure, the detailed description will be omitted.

Terms such as first and second may be used to describe various components, but the components should not be limited by the terms. The terms are used only for the purpose of distinguishing one component from another.

The terms used in the present application are used only to describe a particular embodiment and are not intended to limit the present disclosure. Singular expressions include plural expressions unless the context clearly dictates otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail based on embodiments of the present disclosure. Like reference numerals presented in each figure indicate like members.

FIG. 2 is a diagram for describing a schematic system configuration for implementing a method of analyzing an output of a neural network in accordance with an embodiment of the present disclosure.

Referring to FIG. 2, an output analysis system 100 according to the technical idea of the present disclosure may be installed in a predetermined server 10 to implement the technical idea of the present disclosure. An average expert in the art of the present disclosure will be able to easily infer that the server 10 refers to a data processing device having a calculating ability for implementing the technical ideas of the present disclosure, and in general, any device capable of performing specific services, such as a personal computer and a mobile terminal, as well as a data processing device that is accessible to a client through a network, may be defined as a server.

The output analysis system 100 may be implemented to receive an output result that is output by the neural network 200 from the pre-trained neural network 200.

The output analysis system 100 may be configured to perform wired and wireless communication with the neural network 200 or receive the output result through a predetermined protocol such as a function call.

The neural network 200 may have been trained to output a probability value for whether the disease is developed for a certain disease. The neural network 200 may be configured to output a numerical value, that is, the probability value, indicating the likelihood whether the disease is developed for each pixel on a biometric image.

The technical idea for implementing the neural network 200 for performing this function is disclosed in detail in the prior art applied by the present applicant, the Korean patent registration number (102172213, Two-face disease diagnosis system and method therefor), the registration number (101944536, System for diagnosing disease using neural network and method therefor), and thus detailed descriptions will be omitted in this specification.

The server 10, in which the neural network 200 and the output analysis system 100 are included, may be a system, that is, a diagnostic system for diagnosing a disease. The neural network 200 and the output analysis system 100 may be provided in one physical device, that is, the server 10, but may be provided in each different physical devices according to an embodiment, and an average expert skilled in the art of the present disclosure will be able to easily infer that various modifications may be made as needed.

The diagnostic system 10 may be configured to communicate with certain terminals 20, 20-1 to train the neural network 200.

The terminals 20, 20-1 may be configured to transmit a plurality of training data to the neural network 200 to train the neural network 200, and the neural network 200 may be trained using the received training data.

The neural network 200 may include a convolutional neural network (CNN) that is widely known in the deep learning field, but is not limited thereto.

In addition, the terminals 20, 20-1 may be configured to output a biometric image which is a diagnosis target to the diagnostic system 10 in order to diagnose a disease using the trained neural network 200.

The neural network 200 may then transmit an output result including a probability value for whether the disease is developed for each pixel for the biometric image to the output analysis system 100, and the output analysis system 100 may analyze the received output result according to the technical idea of the present disclosure and output the analysis result. The output analysis result may be transmitted to the terminals 20, 20-1.

The analysis result may be a result derived by distinguishing a lesion area in the biometric image. The analysis result may include information on whether to be included in the lesion area by each pixel.

The output analysis system 100 according to the technical idea of the present disclosure may be configured to receive the output result from the neural network 200 and analyze the received output result to output the analysis result through predetermined criteria so as to match with the actual disease state (whether the disease is developed and/or the pixels where the disease is developed, that is, the lesion area) as much as possible.

It is apparent that, for the actual disease state, annotation information annotated by a specialist may be used.

To this end, the output analysis system 100 needs to determine which reference value to use for each pixel to determine whether the disease is developed in the pixel if it is greater than or equal to the value.

In other words, according to the technical idea of the present disclosure, rather than determining whether the disease is developed for each pixel using a reference value arbitrarily determined in advance depending on the user's will, a process of determining so that the reference value is consistent with the actual disease state as much as possible using a plurality of output results of the trained neural network 200 may be gone through. The reference value determined by this process may be defined as an optimal reference value herein.

After going through the process of determining the optimal reference value, the output analysis system 100 may determine whether the pixel is included in the lesion area for each pixel using the determined optimal reference value, when receiving the output result of the neural network 200 corresponding to the biometric image which is an actual diagnosis target.

In addition, according to the technical idea of the present disclosure, the output analysis system 100 not only uses the optimal reference value, but also presets the criteria to determine whether the lesion area detected by the optimal reference value is actually a valid lesion area. These criteria may be defined herein as cut-off values.

In other words, according to the technical idea of the present disclosure, the output analysis system 100 may be configured to detect the lesion area by analyzing the output result of the neural network 200 in the biometric image which is a diagnosis target using the optimal reference value, and determine once again whether the detected lesion area is an actually valid lesion area, to finally decide the lesion area in the biometric image.

This may be because the neural network 200 has been trained to make the sum of errors be minimized in the entire biometric image through the probability value for whether the disease is developed for each pixel, and in this case, the error may be small for each pixel when the output result of the neural network 200 is used as it is. However, when detecting the lesion area using the output result of the neural network 200 as it is (i.e., using the determination of whether a disease is developed for each pixel), it is highly likely that the result may be inconsistent with the presence of the disease for an actual sample (for each biometric image).

This is because, even if the disease is highly likely to be developed in a very local area (e.g., an area corresponding to a specific pixel), the disease may not actually be developed, but whether the actual disease develops is likely to be determined in consideration of both the state of the area and the peripheral area, rather than being determined only by the individual state of the local area corresponding to a specific pixel.

Therefore, when determining whether the disease is developed for each pixel using the probability values of whether a disease is developed for each pixel to decide the lesion area using only the result, even when determining by optimizing the optimal reference value, there is always a probability that the decided lesion area is actually an area where the disease is not developed.

Therefore, according to the technical idea of the present disclosure, not only the optimal reference value may be determined so that whether the disease is developed for each pixel detected as an analysis result of the output analysis system 100 is consistent with the actual state (annotated information) as much as possible, but also the optimal cut-off value may be determined so that the lesion area detected using the determined optimal reference value is also consistent with the actual state (annotated information) as much as possible.

The optimal cut-off value may be used as a criterion to determine whether the detected lesion area is actually a valid lesion area using the optimal reference value, and the optimal cut-off value may be a criterion for the number of pixels included in the detected lesion area.

The output analysis system 100 may be configured to primarily detect the lesion area using the optimal reference value and decide the lesion area as a valid lesion area only when the number of pixels included in the detected lesion area is greater than or equal to the optimal cut-off value.

Ultimately, according to the technical idea of the present disclosure, the output analysis system 100 may go through a process of determining a criterion for analyzing the output result of the trained neural network 200 to be consistent with the actual state as much as possible, apart from the training of the neural network 200.

In addition, such criterion may include an optimal reference value for the analysis result for each pixel (determination result for whether the disease is developed for each pixel) to be consistent with the actual state as much as possible and/or an optimal cut-off value for the lesion area detected using the optimal reference value to be consistent with the actual state as much as possible.

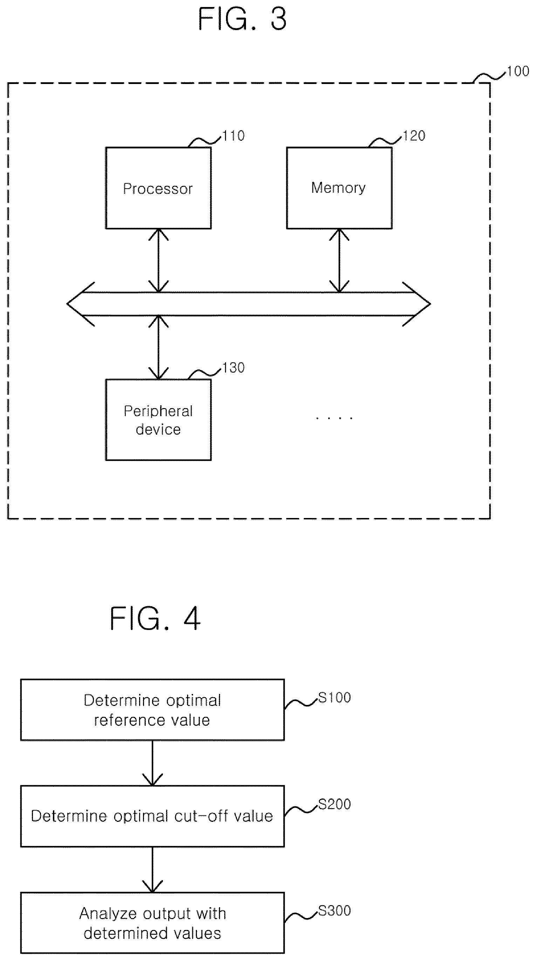

The output analysis system 100 for implementing this technical idea may have a configuration as shown in FIG. 3.

FIG. 3 is a diagram for describing a schematic configuration of the output analysis system of the neural network in accordance with an embodiment of the present disclosure.

The output analysis system 100 may be provided with a memory 120 in which a program for implementing the technical idea of the present disclosure is stored, and a processor 110 configured to execute the program stored in the memory 120.

An average expert in the art of the present disclosure may easily infer that the processor 110 may be named by various names such as a CPU and a mobile processor according to an embodiment of the output analysis system 100. In addition, an average expert in the art of the present disclosure may easily infer that the output analysis system 100 may be implemented as a plurality of physical devices are organically combined, and in this case, at least one processor 110 may be provided for each physical device to implement the output analysis system 100.

The memory 120 may be implemented as any type of storage device in which the program is stored and accessible by the processor to run the program. In addition, the memory 120 according to an embodiment in a hardware form may be implemented as a plurality of storage devices rather than any single storage device. In addition, the memory 120 may include not only a main memory device but also a temporary memory device. It may also be implemented as a volatile memory or a non-volatile memory, and may be defined in the sense of including any type of information storage means implemented so that the program may be stored and run by the processor.

The program, as described below, may be a set of a series of codes to determine an optimal reference value and/or an optimal cut-off value according to the technical idea of the present disclosure. In addition, the program, as described below, may determine each different reference value for each pixel, that is, reference values for each pixel.

In addition, when the biometric image which is a target of diagnosis is input to the diagnostic system 10, the program may receive an output result corresponding to the biometric image from the neural network 200, and analyze the received output result using the determined optimal reference value, optimal cut-off value, and/or reference value for each pixel to output the analysis result.

According to an embodiment, the output analysis system 100 may be a system configured to be operated and implemented by a subject (e.g., medical institution, etc.) who intends to diagnose a disease using the neural network 200 according to the technical idea of the present disclosure, may be implemented in various ways such as a web server or a computer, and may be defined as including any type of data processing device capable of performing functions defined in this specification.

In addition, the output analysis system 100 may be provided in the same physical device as the neural network 200 or may be provided in different physical devices. The output analysis system 100 and the neural network 200 may be combined to be implemented as a diagnostic system 10 to perform a diagnosis.

In addition, various peripheral devices 130 may further be provided according to an embodiment of the output analysis system 100. For example, an average expert in the art of the present disclosure will easily infer that peripheral devices such as a keyboard, a monitor, a graphics card, a communication device, and a scanner may be further included in the output analysis system 100.

Hereinafter, an average expert in the art of the present disclosure may easily infer that a case in which the output analysis system 100 performs a predetermined function refers to a case in which the processor 110 runs the program stored in the memory 120 to perform the function.

FIG. 4 is a flowchart for outlining the concept of analyzing an output result of the neural network in accordance with an embodiment of the present disclosure.

Referring to FIG. 4, in order to implement the method of analyzing an output of the neural network according to the technical idea of the present disclosure, the output analysis system 100 may be configured to determine the optimal reference value to analyze the output result of the neural network 200 (S 100).

In addition, though the lesion area may be simply detected using the optimal reference value and the detected lesion area detected may be determined as the final lesion area, as described above, the output analysis system 100 may further determine the optimal cut-off value to determine whether the detected lesion area detected using the determined optimal reference value is valid (S200).

In this way, if the optimal reference value and/or the optimal cut-off value are determined, the output analysis system 100 may then analyze, when an output result corresponding to a biometric image as a diagnosis target from the neural network 200, the received output result using the predetermined optimal reference value and/or optimal cut-off value, to output the analysis result.

The analysis result may be information on whether the disease is developed and/or lesion area (e.g., location, boundary, number of pixels, etc.) for each pixel that is finally determined. Surely, even if it is primarily determined that a specific pixel is included in the lesion area using the optimal reference value, when the lesion area is determined as being invalid through the optimal cut-off value, it is apparent to be finally determined that the primary determination is invalidated for the specific pixel and not included in the lesion area (the disease is not developed).

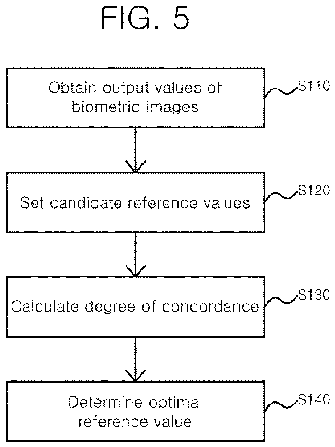

The process of determining the optimal reference value may be as shown in FIG. 5.

FIG. 5 is a flowchart for describing a way of determining an optimal reference value in accordance with an embodiment of the present disclosure.

Referring to FIG. 5, first, the output analysis system 100 may be configured to obtain an output result including an output value for each pixel for each of the plurality of biometric images from the neural network 200 (S 110).

The plurality of biometric images may be biometric images that are not used for training the neural network 200. In addition, the plurality of biometric images may all be annotated.

The output analysis system 100 may then set a plurality of candidate reference values (S120). A plurality of candidate reference values may be set to have a difference, such as a preset unit interval (e.g., 0.01, 0.001, etc.).

The output analysis system 100 may then calculate the degree of concordance for each of the set candidate reference values. The degree of concordance may be information indicating the degree in which the determination result of the output analysis system 100 for each pixel is consistent with the actual state (i.e., annotated result) for the plurality of biometric images.

To this end, the output analysis system 100 may analyze the output results of biometric image using each of the candidate reference values to generate whether the disease is developed for each pixel, that is, an analysis result. For example, if the probability value included in the output result for a specific pixel is greater than or equal to the candidate reference value, it may be determined to be included in the lesion area, otherwise it may be determined not to be included in the lesion area.

In addition, it is possible to calculate the degree of concordance using the generated analysis result for each pixel, and the degree of concordance may be determined according to the degree to which the detection result detected by applying the candidate reference value for each pixel is consistent with the annotated result.

The degree of concordance may be defined in various ways.

According to an example, the degree of concordance may be defined using any one of accuracy, Intersection over Union (IoU), or Dice Similarity Coefficient (DSC) or a combination thereof, derived by comparing the determination result for each pixel and the annotated result for all of the plurality of biometric images.

Alternatively, the degree of concordance may be defined using any one of an average of accuracy, an average of an Intersection over Union (IoU), or an average of a Dice Similarity Coefficient (DSC) or a combination thereof, derived by each of the plurality of biometric images.

When the output analysis system 100 defines the lesion area detected as a result of analysis by applying each of the candidate reference values as the first lesion area and defines the lesion area included in the pre-annotated information as the second lesion area, TP indicates the number of pixels included in both the first lesion area and the second lesion area, FN the number of pixels included only in the second lesion area, the FP the number of pixels included only in the first lesion area, and TN the number of pixels not included in both the first lesion area and the second lesion area.

The output analysis system 100 may be configured to calculate TP, TN, FP, and FN from the analysis results analyzed using each of the candidate reference values and calculate the accuracy, IoU, and DSC based on the same. Accuracy, IoU, and DSC are widely used as indicators to measure the performance of the trained neural network, and the accuracy may be defined as (TP+TN)/(TP + TN + FP + FN), the IoU as TP/(TP + FP + FN), and the DSC as 2×TP/(2×TP + FP + FN).

It is apparent that the output analysis system 100 may be configured to determine the candidate reference value that has the highest degree of concordance as the optimal reference value based on the degree of concordance of each of the candidate reference values.

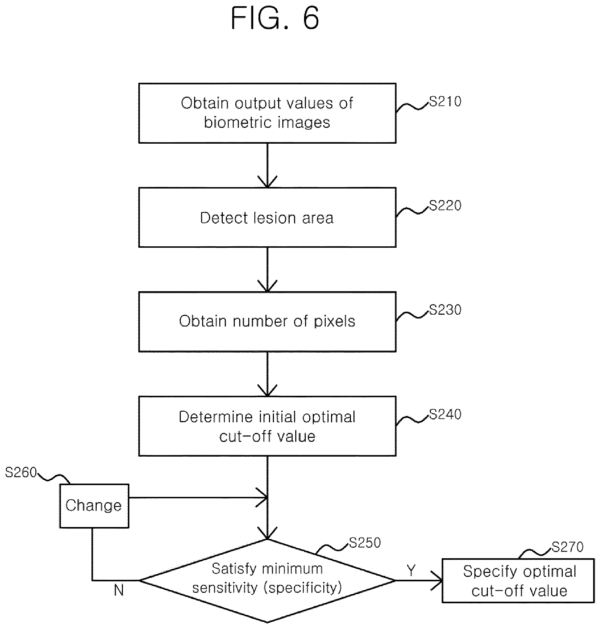

On the other hand, the process of determining the optimal cut-off value may be as shown in FIG. 6.

FIG. 6 is a flowchart for describing a way of determining an optimal cut-off value in accordance with an embodiment of the present disclosure.

Referring to FIG. 6, the output analysis system 100 may be configured to obtain an output result including an output value for each pixel of each of the plurality of biometric images from the neural network 200 (S210). The plurality of biometric images that are used to determine the optimal cut-off value may also be biometric images that are not used for training the neural network 200. Surely, they may be different from the biometric images used to determine the optimal reference value or the same. Each of these biometric images in that case may all be annotated.

Then the output analysis system 100 may be configured to detect the lesion area using a predetermined optimal reference value (S220). In other words, when the probability value determined by the neural network 200 for each pixel is greater than or equal to the optimal reference value, it is possible to detect the lesion area from each of the biometric images by detecting as a pixel not included in the lesion area, otherwise detected as a pixel included in the lesion area.

The lesion area may be a cluster of pixels in which it is determined that a disease is developed, that is, pixels whose probability value is greater than or equal to the optimal reference value, and the output analysis system 100 may calculate the number of pixels included in each of the lesion areas (S230).

In addition, based on the number of pixels calculated, the optimal cut-off value may be determined (S240).

The number of pixels calculated in that case may be summed by each biometric image or by each lesion area. For example, there may be a plurality of lesion areas that are independent (separated) of each other in any one biometric image.

In this case, according to an embodiment, even when a plurality of lesion areas exist in a single biometric image, the number of pixels included in the lesion areas for each biometric image may all be simply summed up to calculate the number of pixels included in the lesion for each of the biometric images. Alternatively, the number of pixels may be calculated individually for each independent lesion area, not for each biometric image.

In the former case, the optimal cut-off value may be determined so that the results of the lesion area detected through the output analysis system 100 are consistent as much as possible with whether the disease is developed in the annotated information based on any one biometric image, and in the latter case, the optimal cut-off value may be determined so that the results of the lesion area detected through the output analysis system 100 are consistent as much as possible with whether the disease is developed in the annotated information based on each of the lesion areas.

In other words, even if the disease is not actually developed, the lesion area may be detected through the output analysis system 100, in which case it may be desirable that the lesion area be cut off.

Thus, the output analysis system 100 may primarily detect the lesion area for a plurality of biometric images and, when a lesion area having pixels less than the optimal cut-off value is invalidated, determine the optimal cut-off value so that the detected result is consistent with the actual disease state as much as possible (for each biometric image or each lesion area).

For example, when determining the optimal cut-off value by summing the number of pixels based on the biometric image, even if there are a plurality of lesion areas detected in a specific biometric image, the number of pixels included in each of the lesion areas may be simply summed, and the optimal cut-off value may be determined so that the summed number of pixels is consistent with whether the disease is developed in the specific biometric image. For example, the output analysis system 100 may determine the optimal cut-off value through a predetermined algorithm, and in this case, if the disease is not developed in the specific biometric image, an algorithm may be designed so that a value that is greater than the summed number of pixels is likely to be determined as the optimal cut-off value, and, if the disease is developed in the specific biometric image, an algorithm may be designed so that a value less than the summed number of pixels is likely to be determined as the optimal cut-off value.

For example, when determining the optimal cut-off value by summing the number of pixels based on each lesion area, the optimal cut-off value may be determined so that whether the disease is developed in the lesion area may be consistent with the number of pixels for each of the detected lesion areas. For example, the output analysis system 100 may determine the optimal cut-off value through a predetermined algorithm, and in this case, if the disease is not actually developed in the specific lesion area detected by the output analysis system 100, an algorithm may be designed so that a value greater than the number of pixels in the specific lesion area is likely to be determined as the optimal cut-off value, and if the disease is actually developed in the specific lesion area, an algorithm may be designed so that a value less than the number of pixels in the specific lesion area is more likely to be determined as the optimal cut-off value.

The determination of this optimal cut-off value may be defined as a process of searching for an optimal value so that the detection result of the output analysis system 100 when the optimal cut-off value is applied for each of the biometric images (each of the lesion areas) may be consistent as much as possible with the actual disease state, and this process may be defined as a one-dimensional (linear) optimization problem.

This optimization problem may be defined as a linear regression, logistic regression, or support vector machine problem, as is widely known, and an average expert in the art of the present disclosure may easily infer that the optimal cut-off value may be determined using at least one of these algorithms.

On the other hand, in a system for determining the disease, there may be a case in which the system must be designed to satisfy the sensitivity or specificity to be satisfied at least. Sensitivity may refer to a rate obtained by detecting an area where the actual disease is developed as the disease is developed, while specificity to the rate obtained by detecting an area where the actual disease is not developed as the disease is not developed, and there are cases in which sensitivity is prioritized or specificity is prioritized depending on the type of disease.

Accordingly, the output analysis system 100 may determine the optimal cut-off value so that the preset minimum sensitivity or specificity is satisfied.

To this end, the output analysis system 100 may determine the optimal cut-off value in the same manner first as described above, and the optimal cut-off value thus determined may become an initial optimal cut-off value (S240).

The output analysis system 100 may then determine whether the current optimal cut-off value satisfies the minimum sensitivity or the minimum specificity (S250), and if the determination result is satisfactory, the current optimal cut-off value may be specified as the final optimal cut-off value (S270).

When the current optimal cut-off value does not satisfy the minimum sensitivity or minimum specificity as a result of the determination (S250), the current optimal cut-off value is changed only at a predetermined unit interval (e.g., 1, 5, etc.) (S260), and the process of determining whether the changed optimal cut-off value satisfies the minimum sensitivity or minimum specificity may be repeated to finally specify the optimal cut-off value that satisfies the minimum sensitivity or the minimum specificity (S270).

Surely, the number of cut-offs may be reduced by a predetermined unit interval from the current optimal cut-off value in order to satisfy the minimum sensitivity, and, in order to satisfy the minimum specificity, the number of cut-offs may be increased by a predetermined unit interval from the current optimal cut-off value to determine whether the changed optimal cut-off value satisfies the minimum sensitivity or the minimum specificity.

On the other hand, the optimal reference value as described above may be uniformly applied for all pixels included in the biometric image to be a criterion to determine whether to be included in the lesion area for each pixel, and according to the technical idea of the present disclosure, these reference values may be differently set for each pixel.

This is because, when the same reference value (optimal reference value) is applied to all pixels, a result that is completely different from the detection result of the peripheral pixels may be derived, and in general, the detection result of a specific pixel is closely related to the detection result of the peripheral pixel, such that it is more desirable to correct the same. For example, there may be a case in which it is determined that a disease is all developed for the peripheral pixels of the specific pixel while it is not determined that a disease is not developed in the specific pixel, and, in this case, it is likely to have difference in the state of development of the actual disease.

Therefore, according to the technical idea of the present disclosure, it is necessary to determine whether the disease for each pixel is developed by applying different reference values for each pixel, and for this purpose, the output analysis system 100 first determines the optimal reference value as described in FIG. 5, and then determine the reference value for each pixel by making correction in consideration of the probability value of the peripheral pixel.

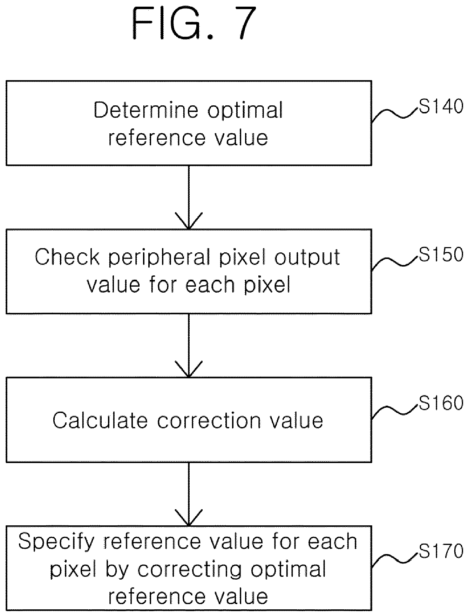

An example thereof will be illustrated with reference to FIG. 7.

FIG. 7 is a flowchart for describing a way of determining a reference value for each pixel in accordance with an embodiment of the present disclosure.

Referring to FIG. 7, the output analysis system 100 may be configured to determine the optimal reference value to be commonly used for the analysis of biometric images first as shown in FIG. 5 (S140).

In addition, the optimal reference value may be corrected for each pixel to specify the reference value for each pixel (S170).

In this case, for the correction value for correcting the optimal reference value for each pixel, a probability value (i.e., the output value of the neural network 200) of the peripheral pixel of the pixel may be used.

For example, the output analysis system 100 may set up a window including the specific pixel to determine a reference value for each pixel of a specific pixel (target pixel). The window may have a preset size (e.g., 3 by 3, 5 by 5, etc.).

The output analysis system 100 may then specify other pixels included in the window as peripheral pixels of the specific pixel and identify the output value of the neural network 200 of the other pixels (S 150).

In addition, based on the output value of the neural network 200 of the other pixels, a correction value to be applied to the specific pixel may be calculated (S 160). The correction value may be determined in various ways.

For example, the standard deviation of the output values of the specific pixel and other pixels or the value obtained by dividing the standard deviation by a certain value may be the correction value.

The output analysis system 100 may then specify the reference value for each pixel of the specific pixel by adding or subtracting the correction value from the optimal reference value (S170).

When the reference value for each pixel is specified in this way, the probability value of the disease development of the peripheral pixels affects the determination of whether the disease is developed in the specific pixel, thereby having an effect that the probability for the determination result of the specific pixel to become completely different from the determination of the peripheral pixels.

On the other hand, the method of analyzing an output according to an embodiment of the present disclosure may be implemented in the form of a computer-readable program command and stored on a computer-readable recording medium, and a control program and a target program according to an embodiment of the present disclosure may also be stored in a computer-readable recording medium. A computer-readable recording medium includes all types of recording devices in which data that may be read by a computer system is stored.

Program commands recorded on a recording medium may be specifically designed and configured for the present disclosure or may be known and available to those skilled in the software field.

Examples of a computer-readable recording medium include magnetic media such as hard disks, floppy disks, and magnetic tapes, optical media such as CD-ROMs and DVDs, magneto-optical media such as floptical disks, and hardware devices specifically configured to store and execute program commands such as ROMs, RAM, and flash memory. In addition, the computer-readable recording medium is distributed in computer systems connected through a network, so that computer-readable codes may be stored and executed in a distributed manner.

Examples of program commands include machine codes such as those generated by compilers, as well as devices that electronically process information using interpreters, such as high-level language code that may be executed by a computer.

The above-described hardware device may be configured to operate as one or more software modules to perform the operation of the present disclosure, and vice versa.

The foregoing description of the present disclosure is for illustrative purposes only, and a person skilled in the art to which the present disclosure pertains will be able to understand that it may be easily transformed into other concrete forms without changing the technical idea or essential features of the present disclosure. Therefore, the embodiments described above should be understood as exemplary and not limited in all respects. For example, each component described as a single type may be implemented in a distributed form, and likewise components described as a distributed form may be implemented in a combined form.

The scope of the present disclosure is indicated by the claims to be described later rather than by the above detailed description, and the meaning and scope of the claims and all modifications or modified forms derived from the concept of equivalence thereof should be construed as included in the scope of the present disclosure.

### Industrial Applicability

The present disclosure may be used for a method of analyzing an output of a neural network and a system therefor.

## Claims

1. A method of analyzing an output of a neural network that analyzes an output result of the neural network trained to output a probability of disease development by each pixel of a biometric image, the method comprising:
determining, by an output analysis system, an optimal reference value, which is a criterion to detect whether a disease is developed in each pixel depending on whether an output result value of the neural network for the pixel is greater than or equal to the optimal reference value;
determining, by the output analysis system, an optimal cut-off value to determine, for a detected lesion area on the biometric image that is specified when whether the diseases is developed for each pixel is detected for the biometric image using the determined optimal reference value, whether the detected lesion area is valid; and
when receiving an output result corresponding to a diagnostic biometric image to be diagnosed from the neural network, performing, by the output analysis system, output analysis on the output result using the determined optimal reference value and the determined optimal cut-off value.

2. The method of claim 1, wherein the determining of the optimal reference value comprises:
obtaining, by the output analysis system, an output value for each pixel of each of a plurality of biometric images using the neural network;
setting, by the output analysis system, a plurality of candidate reference values and calculating, for each of the set candidate reference values, a degree of concordance when the disease development is detected by applying each of the candidate reference values, wherein the degree of concordance is determined by a degree of which a detection result detected by applying the candidate reference value for each pixel and an annotated result are concordant; and
determining, by the output analysis system, the optimal reference value based on the calculated degree of concordance of each of the candidate reference values.

3. The method of claim 2, wherein the calculating of the degree of concordance when whether the disease is developed is determined by applying each of the candidate reference values comprises calculating the degree of concordance using at least one of
accuracy, Intersection over Union (IoU), or Dice Similarity Coefficient (DSC) for all of the plurality of biometric images; or
an average of accuracy, Intersection over Union (IoU), or Dice Similarity Coefficient (DSC) of each of the plurality of biometric images, and
wherein the accuracy is defined as (TP+TN)/(TP + TN + FP + FN), the IoU as TP/(TP + FP + FN), and the DSC as 2×TP/(2×TP + FP + FN), and
the TP is the number of pixels included in both a first lesion area detected through the output analysis system and an annotated second lesion area, the FN is the number of pixels included only in the second lesion area, the FP is the number of pixels included only in the first lesion area, and the TN is the number of pixels not included in both the first lesion area and the second lesion area.

4. The method of claim 1, wherein the determining of the optimal cut-off value to determine whether the detected lesion area is valid comprises:
obtaining, by the output analysis system, an output value for each pixel of each of a plurality of biometric images using the neural network;
detecting, by the output analysis system, at least one first lesion area included in each of the plurality of biometric images using the obtained output value for each pixel of each of the plurality of biometric images and the optimal reference value to obtain the number of pixels included in the first lesion area; and
determining, based on the number of pixels for each of the detected at least one first lesion area and whether the disease is developed actually, the optimal cut-off value which is a criterion to determine the validity of the detected first lesion area.

5. The method of claim 4, wherein the determining of the optimal cut-off value which is the criterion to determine the validity of the detected first lesion area based on the number of pixels included in a second lesion area which is an annotated lesion area for each of the plurality of biometric images and the first lesion area comprises determining, by the output analysis system, the optimal cut-off value using any one of linear regression, logistic regression, or support vector machine.

6. The method of claim 4, wherein the determining of the optimal cut-off value which is the criterion to determine the validity of the detected first lesion area based on the number of pixels included in a second lesion area which is an annotated lesion area for each of the plurality of biometric images and the first lesion area comprises:
determining, by the output analysis system, an initial optimal cut-off value;
determining, by the output analysis system, whether the initial optimal cut-off value satisfies a predetermined minimum sensitivity or a predetermined minimum specificity; and
if the determination result is satisfied, determining the initial optimal cut-off value as the optimal cut-off value and, if unsatisfied, searching for the optimal cut-off value while sequentially changing the initial optimal cut-off value by a predetermined unit until the minimum sensitivity or the minimum specificity is satisfied.

7. The method of claim 1, further comprising:
determining, by the output analysis system, a reference value for each pixel by each pixel using the determined optimal reference value,
wherein the output analysis system detects whether the disease is developed for each pixel for the diagnostic biometric image to be diagnosed using the determined reference value for each pixel, and
the reference value for each pixel is determined by correcting the optimal reference value using an output value of the neural network for at least one predetermined peripheral pixel based on a target pixel.

8. A method of analyzing an output of a neural network that analyzes an output result of the neural network trained to output a probability of disease development by each pixel of a biometric image, the method comprising:
obtaining, by the output analysis system, an output result value of the neural network by each pixel for a diagnostic biometric image to be diagnosed; and
detecting, by the output analysis system, whether a disease is developed for each pixel by comparing the obtained output result value with a predetermined reference value,
wherein the reference value is determined differently for each pixel and determined by correcting entire reference values determined in a predetermined manner for the entire diagnostic biometric image using the output result value of the neural network for at least one peripheral pixel predetermined based on a target pixel.

9. A computer program installed in a data processing device and recorded on a non-transitory medium for implementing the method according to any one of claims 1 to 8.

10. An output analysis system of a neural network, the output analysis system comprising:
a processor; and
a memory in which a neural network trained to output a probability of disease development by each pixel of a biometric image and a program are stored,
wherein the processor is configured to run the program to:
determine an optimal reference value, which is a criterion to detect whether a disease is developed in each pixel depending on whether an output result value of the neural network for the pixel is greater than or equal to the optimal reference value;
determine an optimal cut-off value to determine, for a detected lesion area on the biometric image that is specified when whether the diseases is developed for each pixel is detected for the biometric image using the determined optimal reference value, whether the detected lesion area is valid; and
when receiving an output result corresponding to a diagnostic biometric image to be diagnosed from the neural network, perform output analysis on the output result using the determined optimal reference value and the determined optimal cut-off value.

11. The output analysis system of claim 10, wherein the processor is configured to run the program to:
obtain an output value for each pixel of each of a plurality of biometric images using the neural network;
set a plurality of candidate reference values and calculate, for each of the set candidate reference values, a degree of concordance when the disease development is detected by applying each of the candidate reference values, wherein the degree of concordance is determined by a degree of which a detection result detected by applying the candidate reference value for each pixel and an annotated result are concordant; and
determine the optimal reference value based on the calculated degree of concordance of each of the candidate reference values.

12. The output analysis system of claim 10, wherein the processor is configured to run the program to:
obtaining an output value for each pixel of each of a plurality of biometric images using the neural network;
detect at least one first lesion area included in each of the plurality of biometric images using the obtained output value for each pixel of each of the plurality of biometric images and the optimal reference value to obtain the number of pixels included in the first lesion area; and
based on the number of pixels for each of the detected at least one first lesion area and whether the disease is developed actually, determine the optimal cut-off value which is a criterion to determine the validity of the detected first lesion area.

13. The output analysis system of claim 12, wherein the processor is configured to run the program to determine an initial optimal cut-off value, determine whether the initial optimal cut-off value predetermined satisfies a predetermined minimum sensitivity or a predetermined minimum specificity, and if the determination result is satisfied, determine the initial optimal cut-off value as the optimal cut-off value and, if unsatisfied, search for the optimal cut-off value while sequentially changing the initial optimal cut-off value by a predetermined unit until the minimum sensitivity or the minimum specificity is satisfied.

14. The output analysis system of claim 10, wherein the processor is configured to run the program to:
determine a reference value for each pixel by each pixel using the determined optimal reference value; and
detect whether the disease is developed for each pixel for the diagnostic biometric image to be diagnosed using the determined reference value for each pixel,
wherein the reference value for each pixel is determined by correcting the optimal reference value using an output value of the neural network for at least one predetermined peripheral pixel based on a target pixel.

15. An output analysis system of a neural network, comprising:
a processor; and
a memory in which a neural network trained to output a probability of disease development by each pixel of a biometric image and a program are stored,
wherein the processor is configured to run the program to:
obtain an output result value of the neural network by each pixel for a diagnostic biometric image to be diagnosed; and
detect whether a disease is developed for each pixel by comparing the output result value with a predetermined reference value, and
wherein the reference value is determined differently for each pixel and determined by correcting entire reference values determined in a predetermined manner for the entire diagnostic biometric image using the output result value of the neural network for at least one peripheral pixel predetermined based on a target pixel.
